# EUROPÄISCHE PATENTSCHRIFT

(11) **EP 3 873 373 B1**
(45) Veröffentlichungstag und Bekanntmachung des Hinweises auf die Patenterteilung: **21.08.2024**
(21) Anmeldenummer: 19798016.2
(22) Anmeldetag: 28.10.2019
(51) Int. Cl.: A61B 50/30, A61L 2/26, B21D 53/48, B21D 22/04, A61B 50/33, A61B 50/00, A61B 90/00, B65D 21/02

(54) **BEHÄLTERSYSTEM UND VERFAHREN ZUR AUSBILDUNG EINES KNAUFS IN EINEM BEHÄLTER**
CONTAINER SYSTEM AND METHOD FOR FORMING A KNOB IN A CONTAINER
SYSTÈME DE RÉCIPIENT ET PROCÉDÉ POUR LA FORMATION D'UNE POIGNÉE DANS UN RÉCIPIENT

(30) Priorität: 29.10.2018 DE 102018126975
(43) Veröffentlichungstag der Anmeldung: 08.09.2021
(73) Patentinhaber: Aesculap AG, 78532 Tuttlingen (DE)
(72) Erfinder: HÖFLER, Martina, 78647 Trossingen (DE); ZIERIS, Gerold, 78532 Tuttlingen-Möhringen (DE); SCHWEIZER, Matthias, 78532 Tuttlingen (DE)
(74) Vertreter: Winter, Brandl - Partnerschaft mbB
(86) Internationale Anmeldenummer: PCT/EP2019/079343
(87) Internationale Veröffentlichungsnummer: WO 2020/089143

(56) Entgegenhaltungen:
- WO-A1-2018/104390
- DE-C- 293 296
- GB-A- 453 055
- KR-B1- 101 195 042
- US-A1- 2017 224 434

## Beschreibung

### Technisches Gebiet

Die vorliegende Erfindung betrifft ein Behältersystem mit einem Behälter, insbesondere einem medizinischen oder chirurgischen Sterilbehälter vorzugsweise mit einem wannenartigen Behältergefäß und/oder einem Behälterdeckel, mit zumindest einem separaten Koppelelement / Koppelbauteil und mit zumindest einem Koppelsystem / Anbindungssystem zur lösbaren Anbindung des Koppelelements an den Behälter, wobei das Koppelsystem einen an dem Behälter ausgebildeten männlichen Koppelabschnitt und einen an dem Koppelelement ausgebildeten weiblichen Koppelabschnitt aufweist, die miteinander koppelbar und entkoppelbar/auskoppelbar sind, wobei der männliche Koppelabschnitt integral in einer Wand des Behälters ausgebildet ist.

Daneben betrifft die Erfindung ein (Umform-)Verfahren zur Ausbildung eines Knaufs in bzw. an einem Behälter, insbesondere einem medizinischen oder chirurgischen Sterilbehälter vorzugsweise mit einem wannenartigen Behältergefäß und/oder einem Behälterdeckel gemäß dem Oberbegriff des nebengeordneten Anspruchs.

In dem Stand der Technik werden unterschiedliche Formen einer Anbindung an einen Behälter bzw. ein Behältersystem mit einem Koppelsystem aufgezeigt. Ein lösbares Anbindungsystem / Koppelsystem nach bekanntem Stand der Technik stellen beispielsweise Schrauben dar. Diese werden in einen Korpus eines Behälters, wie etwa einem Sterilbehälter, eingeschraubt und fixieren lösbar eine weitere Komponente bzw. ein Koppelelement an dem Sterilbehälter. Insbesondere können auf diese Weise Verschlusssysteme oder Kennzeichnungsschilder angebracht werden. Auch können beispielsweise Klemmen verwendet, welche das anzubindende Element kraftschlüssig klemmen. Die Klemmen müssen jedoch auch an dem Behälter befestigt werden, was üblicherweise durch Nieten geschieht. Des Weiteren sind aus dem Stand der Technik Sicherungssysteme oder Verschlusssysteme mittels Formschluss oder Einschnapphaken, bekannt, welche an dem Behälter befestigt werden.

Ein Problem des Standes der Technik ist jedoch, dass die bekannten Anbindungs- bzw. Koppelsysteme eine Öffnung für eine feste Anbindung benötigen und damit eine Durchtrittsstelle in einem Bauteil verursachen. Diese Durchtrittsstellen sind potentiell keimdurchlässig bzw. keimundicht und sind zudem schwer zu reinigen bzw. zu desinfizieren. Zudem können in die Spalte, bedingt durch den Kappilareffekt, Wasser als auch Blut und andere Körpersekrete hineingezogen werden. Die Spalte sowie weitere schwer reinigbare Geometrien bergen damit die Gefahr einer Neuverkeimung an diesen Stellen, weshalb das Behältersystem nicht den Anforderungen an eine Sterilität gerecht wird. Auch erschwert dies einen Produktionsprozess und erhöht die Produktionskosten.

Zudem ist bei einigen Kopplungsvarianten eine hohe Zahl an Bauteilen erforderlich, welche nach einer Produktion auch in einer Montage zusammengesetzt werden müssen. Diese verursachen einen erhöhten Produktionsaufwand und können vor allem, da sie oft nicht mit den anderen Bauteilen fest verbunden sind, verloren gehen oder vergessen werden. Eine Handhabung ist somit deutlich erschwert, da eine Überprüfung der korrekten Anzahl an Bauteilen, beispielsweise anhand einer Liste, vonnöten ist. Werden zum Beispiel Clipsysteme mit Rastnasen an Siebkörben zum wieder lösbaren Befestigen von Schildern benutzt, welche sich in den Siebstrukturen des Siebkorbes festhaken können, so sind entsprechende Geometrien mit Öffnungen und Hinterschnitten erforderlich.

Die WO 2018/104390 A1 offenbart beispielsweise ein Behältersystem, das ein Koppelsystem mit männlichen und weiblichen Koppelabschnitten aufweist. Bei diesem ist ein Anbauteil mit einem in der Behälterwandung integral ausgebildeten Befestigungsabschnitt formschlüssig befestigbar oder federelastisch arretierbar.

Die DE 293 296 C offenbart eine Vorrichtung zur Erzeugung von Druckknopfteilen mit einem eingeschnürten Hals. Diese Vorrichtung weist hierfür mehrere parallel zueinander angeordnete Stempel auf.

Die US 2017/224434 A1 offenbart anpassbare medizinische Tabletts mit einem Koppelsystem, das männliche Koppelabschnitte und weibliche Koppelabschnitte aufweist.

### Zusammenfassung der Erfindung

Es ist daher die Aufgabe der Erfindung die Nachteile aus dem Stand der Technik zu vermeiden oder zu mindern und insbesondere ein Behältersystem und ein Verfahren zur Herstellung eines solchen Behältersystems mit einem Koppelsystem zur Verfügung zu stellen, mit dem weitere Koppelelemente an einem Behälter lösbar angebracht / befestigt werden können, wobei sich eine solche Anbindung bzw. ein solches Koppelsystem leicht reinigen lassen und eine keimdichte Verbindung ausbilden sollen. Auch soll die Anzahl an benötigten Bauteilen für eine gute Handhabung und kostengünstige Produktion minimiert werden.

Die Aufgabe der Erfindung wird bei einem gattungsgemäßen Behältersystem erfindungsgemäß durch die Merkmale des Anspruchs 1 und hinsichtlich eines gattungsgemäßen (Umform-)Verfahrens zur Ausbildung eines Knaufs erfindungsgemäß durch die Merkmale des Anspruchs 7 gelöst.

Die Aufgabe der Erfindung wird bei einem gattungsgemäßen Behältersystem erfindungsgemäß dadurch gelöst, dass der männliche Koppelabschnitt integral/stoffeinstückig in einer Wand des Behälters ausgebildet/ausgeformt ist und in Form eines vorstehenden Knaufs / eines vorstehenden Pilzes mit einem Hinterschnitt gestaltet ist. Der Knauf hat dabei eine pilzförmige Struktur, die ähnlich eines Pollers oder eines Sektkorkens ausgebildet ist. Entscheidend an dem männlichen Koppelabschnitt ist, dass dieser stoffeinstückig in der Wand des Behälters selber ausgebildet beispielsweise durch Umformen (Tiefziehen) der Behälterwand ist, so dass die Wand des Behälters (Behälterwand) als Sterilgrenze weiterhin aufrecht erhalten ist, durch welche kein Bakterium, kein Virus und auch kein Fluid dringen kann, und kein weiteres Bauteil hergestellt werden muss als auch dass der vorstehende Knauf einen Hinterschnitt ausbildet, um über den ausgebildeten Hinterschnitt weitere Koppelelemente, wie etwa ein Kennzeichnungsschild, an dem Knauf formschlüssig anzubringen. Der Knauf weist insbesondere einen abgerundeten Kopf auf und ist hierdurch auch besonders geeignet, gereinigt bzw. sterilisiert zu werden. Als angekoppelter Zustand wird dabei der Zustand bezeichnet, in dem der männliche Koppelabschnitt und der weibliche Koppelabschnitt im Wirkeingriff miteinander stehen und das Koppelelement damit an den Behälter angekoppelt ist, und als ausgekoppelter Zustand ist der Zustand zu verstehen, in dem der weibliche und männliche Koppelabschnitt nicht mehr in Wirkeingriff miteinander stehen und der Behälter und das Koppelelement nicht gegeneinander fixiert sind sondern frei anordbar. Es wird dem Anwender ein Behältersystem zur Verfügung gestellt, das individuell einsetzbar ist, eine verminderte Verletzungsgefahr aufgrund von insbesondere abgerundeten Kanten aufweist und eine einfache Ankopplung/Montage und Auskopplung/Demontage von Koppelelementen realisiert. Die Ausbildung des Knaufs lässt die Notwendigkeit weiterer separater Bauteile entfallen.

Vorteilhafte Ausführungsformen sind in den Unteransprüchen beansprucht und werden nachfolgend erläutert.

In einer bevorzugten Ausführungsform kann das Koppelsystem in Form eines Druckknopfsystems ausgebildet sein, bei dem der weibliche Koppelabschnitt (Druckknopf) eine kreisrunde Vertiefung aufweist, in welche der Knauf (dessen Knaufkopf) in einem angekoppeltem Zustand hineinsteht / eingedrückt ist, wobei der Hinterschnitt des Knaufs durch den weiblichen Koppelabschnitt federnd/vorgespannt umgriffen / umfasst wird. Dadurch, dass das Koppelsystem als Druckknopfsystem ausgebildet ist und der weibliche Koppelabschnitt den Knauf vorgespannt umgreift / umfasst, wird eine Grenze einer Zugkraft definiert, die vonnöten ist, um den weiblichen Koppelabschnitt von dem männlichen Koppelabschnitt zu lösen. Damit ist für eine Lösung von dem Knauf eine Mindestkraft erforderlich, die entsprechend der Vorspannung eingestellt werden kann, so dass sichergestellt wird, dass sich der weibliche Koppelabschnitt nicht unbeabsichtigt löst.

Vorzugsweise kann der weibliche Koppelabschnitt einen, insbesondere umfänglichen, Hinterschnitt aufweisen, der den Hinterschnitt des Knaufs umgreift und mittels einer Materialelastizität / Eigenelastizität den Knauf lösbar ankoppelt oder der weibliche Koppelabschnitt kann eine separate Feder aufweisen, die den Hinterschnitt des Knaufs umgreift / umfasst, um den Knauf lösbar anzukoppeln. Um die Vorspannung zu realisieren sind unterschiedliche Varianten denkbar. Zum einen kann an dem weiblichen Koppelabschnitt ebenfalls ein nach innen zeigender Hinterschnitt durch beispielsweise eine umlaufende Leiste ausgebildet sein, wobei sich dieser Hinterschnitt unter Krafteinwirkung aufgrund einer Materialelastizität aufweiten kann, um den Innendurchmesser des Hinterschnitts zu vergrößern. Zieht also der Anwender im angekoppelten Zustand den weiblichen Koppelabschnitt von dem männlichen Koppelabschnitt, so presst der Knauf aufgrund der Zugkraft und seiner an seiner Hinter/ Unterseite trichterförmigen Geometrie, den Hinterschnitt auseinander, so dass dieser sich vergrößert und über den Knauf abgezogen werden kann. Zum anderen kann der weibliche Koppelabschnitt eine Feder aufweisen, wobei diese Feder einen Hinterschnitt ausbildet und den Kopf des Knaufs federnd bzw. vorgespannt umgreift. Wird nun im angekoppelten Zustand der weibliche Koppelabschnitt mit einer Zugkraft beaufschlagt, bewirkt die Geometrie des Knaufs, wie vorstehen beschrieben, dass die Feder auseinandergedrückt wird, ein Abstand zwischen den Armen der Feder sich vergrößert und der weibliche Abschnitt letztlich von dem männlichen Abschnitt abgezogen werden kann. Insbesondere kann die Feder zwei sich diametral gegenüberliegende und im Wesentlichen parallel verlaufende Arme aufweisen. Alternativ kann die Feder in Form eines Seegerrings ausgebildet sein, der in eine umlaufende Nut im weiblichen Koppelabschnitt eingefasst ist.

Gemäß einem weiteren, alternativen Aspekt kann der weibliche Koppelabschnitt eine Vertiefung in Form eines Schlüssellochs mit einem runden Einlegeabschnitt und einem Rastabschnitt mit parallelen Hinterschnitten in Form von Führungsleisten aufweisen, die derart ausgestaltet ist, dass der Knauf in dem Einlegeabschnitt aufnehmbar und entnehmbar ist und nach einer Verschiebung des Knaufs in den Rastabschnitt über die Führungsleisten formschlüssig angekoppelt ist.

In einer weiteren alternativen bevorzugten Ausführungsform kann der weibliche Koppelabschnitt in Form von zwei sich diametral gegenüberliegenden Rastnasen ausgebildet sein, welche im angekoppelten Zustand den Knauf umgreifen und so den männlichen Koppelabschnitt ankoppeln. Die Rastnasen können dabei eine definierte Materialelastizität aufweisen, um zusammen mit ihrer Geometrie, insbesondere über einen langen Ausleger, elastisch gestaltet zu sein. So können die Rastnasen mittels Druckkraft auf den Knauf "aufgeklippt" werden und unter Anwendung einer Zugkraft wieder abgezogen werden. Vorzugsweise kann der weibliche Kopplungsabschnitt auch mehrere Rastnasen, insbesondere drei, vier oder fünf Rastnasen aufweisen, die im angekoppelten Zustand den Knauf umgreifen. Insbesondere sind die Rastnasen über den Umfang gleichmäßig verteilt.

Vorzugsweise ist der Behälter ein Sterilcontainer und das Koppelelement ist ein Kennzeichnungsschild oder eine Kennzeichnungsblende oder eine Perforationsfeldabdeckung oder ein Sensor oder ein Identifizierungselement oder ein Halteelement. Der Sensor kann beispielsweise ein Temperatursensor sein, der einen Temperaturverlauf aufzeichnet. Das Identifizierungselement kann ein Identifizierungsetikett / Tag eines Trackingsystems / Rückverfolgungssystems sein, wie etwa ein RFID-Chip eines RFID-Systems, um den Behälter zu identifizieren. Das Halteelement kann beispielsweise eine Halteklammer sein.

Alternativ oder zusätzlich kann der Behälter ein Siebkorb sein und das Koppelelement ein Kennzeichnungsschild oder eine Kennzeichnungsblende sein. Insbesondere weisen der Sterilcontainer und der Siebkorb die gleiche Geometrie des Knaufs auf, so dass die Koppelemente, wie das Kennzeichnungsschild sowohl an den Sterilcontainer als auch an den Siebkorb ankoppelbar sind. Somit wird dem Anwender ein modulares Behältersystem ähnlich eines Baukastens zur Verfügung gestellt, welches es ermöglicht, die Koppelelemente mit verschiedenen Behältern zu verwenden.

Gemäß einem weiteren Aspekt der Erfindung kann das Behältersystem als Koppelelement einen zweiten Behälter aufweisen, so dass der erste Behälter sich in den zweiten Behälter "einhaken" und ankoppeln kann. Ist nun also an einer Behälterwand der nach außen von dem Behälter hin vorstehende Knauf ausgebildet und auf einer gegenüberliegenden Außenseite des Behälters der komplementäre weibliche Arretierabschnitt, beispielsweise in Form einer nach Innen des Behälters umgeformten Einbuchtung, so lassen sich zwei oder auch mehr Behälter ineinander einhaken.

Vorzugsweise kann der Behälter Metall bzw. einen Metall-Werkstoff, insbesondere Aluminium, oder eine Metall-Legierung aufweisen. Metalle lassen sich besonders gut sterilisieren, sind beständig und halten in der Praxis auftretenden Belastungen, wie etwa Temperaturschwankungen, besonders gut Stand. Auch lassen sie sich gut verarbeiten und sind kostengünstig. Bei Metall-Legierungen kann man je nach Anwendung eine optimale Zusammensetzung der Metalle bzw. metallischer Werkstoffe festlegen, um insbesondere hinsichtlich Festigkeit und chemischer Beständigkeit einen bestmöglichen Werkstoff für den Behälter zu erhalten. Aluminium als Material ist besonders vorteilhaft, da es leicht ist, korrosionsbeständig und zudem gut sterilisierbar. Auch vergüteter Stahl mit entsprechenden Legierungsgehalten oder Edelstahl sind alternative Metall-Werkstoffe für den Behälter. Als Legierungsmetalle für die Metall-Legierung können insbesondere Chrom und/oder Mangan und/oder Nickel und/oder Molybdän und/oder Titan und/oder Niob und/oder Vanadium und/oder Kobalt Verwendung finden.

Gemäß einem weiteren Aspekt der Erfindung kann der Metall-Werkstoff oder die Metall-Legierung zumindest 80 Gewichtsprozent (gew%), besonders bevorzugt 90 Gewichtsprozent, des Behälters ausmachen und insbesondere kann der gesamte Behälter aus dem Metall-Werkstoff oder der Metall-Legierung hergestellt sein. Insbesondere kann der medizinische oder chirurgische Sterilcontainer aus dem Metall-Werkstoff oder der Metall-Legierung hergestellt sein.

Vorzugsweise kann der Behälter durch Tiefziehen hergestellt sein. Die Struktureigenschaften des Behälters nach dem Tiefziehen sind besonders vorteilhaft. Auch lassen sich die Behälter kostengünstig und effizient fertigen. Insbesondere ist die Containerwanne tiefgezogen hergestellt bzw. durch Tiefziehen hergestellt.

Die Aufgabe der Erfindung wird hinsichtlich eines gattungsgemäßen (Umform-) Verfahrens erfindungsgemäß durch die folgenden Schritte gelöst: Anordnen von zwei komplementären Pressmatrizen auf gegenüberliegenden Seiten einer Wand des Behälters, wobei eine männliche Pressmatrize der beiden Pressmatrizen einen Vorsprung und eine weibliche Pressmatrize eine komplementäre Matrizen-Vertiefung aufweist; Pressen der komplementären Pressmatrizen gegeneinander und damit Auspressen / Tiefziehen eines in Richtung Außenseite des Behälters vorragenden Napfes; und Stauchen des Napfes, so dass ein Knauf mit einer pilzförmigen Struktur mit einem Kopf und einem umlaufenden Hinterschnitt ausgeformt/ausgebildet wird. Durch die Form der komplementären Pressmatrizen, welche koaxial zueinander angeordnet werden, wird im zweiten Schritt ein Napf /Pressnapf mit einer zylindrischen oder kegelförmigen Umfangswand und einer, vorzugsweise zu der Behälterwand parallelen oder gewölbten, insbesondere sphärisch gewölbten, Stirnwand / Stirnseite erzeugt. Die Umfangswand und die Stirnwand / Stirnseite weisen dabei noch keinen Hinterschnitt auf. Der Hinterschnitt wird erst im dritten Schritt erzeugt, in welchem der Napf gestaucht wird und in welchem sich die Stirnwand sowie der angrenzende Bereich der Umfangswand verbreitert und den Hinterschnitt ausformt. Durch das erfindungsgemäße Verfahren kann also in der Behälterwand selbst ein Knauf ausgeformt werden, so dass eine Sterilgrenze erhalten bleibt und zudem eine Anzahl an Bauteilen minimiert wird.

In einer bevorzugten Variante kann der Schritt "Stauchen des Napfes" durch ein Werkzeug mit einer planen oder sphärischen und vorzugsweise rotationssymmetrischen Matrizenform/Stempelform erfolgen, welche den Napf plattdrückt, um durch plastische Verformung den Hinterschnitt zu formen. Die Matrizenform kann vorzugsweise eine mehreckige Kontur aufweisen bzw. im Querschnitt gesehen einen mehreckigen Umfang. Insbesondere weist die Matrizenform eine sechseckige oder achteckige Kontur auf.

Gemäß einer alternativen Variante kann der Schritt "Stauchen des Napfes" die Schritte aufweisen: Wechsel der weiblichen Pressmatrize mit der männlichen Pressmatrize; und Pressen der komplementären Pressmatrizen gegeneinander. Durch den Wechsel der beiden Pressmatrizen gegeneinander kann mittels des Vorsprungs der männlichen Pressmatrize der Napf gestaucht werden. Eine Anzahl benötigter unterschiedlicher Werkzeuge kann minimiert werden.

Gemäß einer weiteren alternativen Variante kann der Schritt "Stauchen des Napfes" die Schritte aufweisen: Pressen des Napfes durch ein Formwerkzeug mit einer definierten umfänglichen Knauf-Matrizenkontur, in welches sich der Napf plastisch einpresst und anformt; und Teilen des Formwerkzeugs und entformen des Knaufs. In dieser Variante weist das Formwerkzeug eine hinterschnittige Formgebung auf. Durch den aufgebrachten Druck wird der Napf, ähnlich wie beim Spritzgießen dennoch in diese hinterschnittige Form eingepresst und legt sich an die Form des Formwerkzeugs an. Nach dem Pressvorgang wird das zumindest zweiteilige Formwerkzeug geteilt und das Formwerkzeug kann von dem männlichen Koppelabschnitt entfernt werden. Dieser Aspekt der Erfindung ermöglicht eine spezielle und individuelle Formgebung des Kopfes des Knaufs.

Gemäß einem Aspekt der Erfindung können die Schritte Pressen der komplementären Pressmatrizen gegeneinander und Stauchen des Napfes in einem Press-Prägeschritt (im Wesentlichen simultan) ausgeführt werden, bei welchem die beiden Pressmatrizen gegeneinander gepresst werden, wobei die weibliche Pressmatrize ein Formwerkzeug mit einer definierten umfänglichen Knauf-Matrizenkontur als Prägeform ist, in welches sich der Napf plastisch einpresst und anformt; und Teilen des Formwerkzeugs und entformen des Knaufs. In dieser Variante weist das Formwerkzeug als weibliche Pressmatrize eine hinterschnittige Formgebung auf und durch eine einzige (translatorische) Press-Präge-Bewegung als Press-Prägeschritt, nämlich das Pressen der beiden Pressmatrizen gegeneinander, wird zunächst der Napf über den Vorsprung der männlichen Pressmatrize tiefgezogen und ausgeformt und bei weiterer Druckbeaufschlagung erfolgt aufgrund des Drucks eine plastische Ausformung in das ausgebildete Volumen zwischen den beiden Pressmatrizen, in welches sich der Napf plastisch einpresst, anformt und ein Hinterschnitt ausgebildet wird.

### Kurzbeschreibung der Figuren

Die Erfindung wird nachfolgend anhand bevorzugter Ausführungsformen mithilfe von begleitenden Figuren näher erläutert. Es zeigen:
Fig. 1 eine schematische Querschnittsansicht eines erfindungsgemäßen Behältersystems mit einem Koppelsystem einer ersten bevorzugten Ausführungsform,
Fig. 2 eine perspektivische Ansicht eines Knaufs aus Fig. 1,
Fig. 3 eine schematische Ansicht eines erfindungsgemäßen Behältersystems mit einem Koppelsystem einer weiteren, zweiten bevorzugten Ausführungsform,
Fig. 4 eine Querschnittsansicht des Koppelsystems aus Fig. 3,
Fig. 5 eine Querschnittsansicht eines erfindungsgemäßen Behältersystems mit einem Koppelsystem einer weiteren, dritten bevorzugten Ausführungsform,
Fig. 6 eine perspektivische Ansicht eines Behältersystems mit einem Koppelsystem einer weiteren, vierten bevorzugten Ausführungsform,
Fig. 7 eine perspektivische Ansicht eines Behältersystems mit einem Koppelsystem einer weiteren, fünften bevorzugten Ausführungsform, und
Fign. 8 bis 11 eine Querschnittsansicht der Schritte eines erfindungsgemäßen Umformverfahrens einer ersten bevorzugten Ausführungsform.

Die Figuren sind schematische Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

Nachstehend werden Ausführungsformen der vorliegenden Erfindung unter Zuhilfenahme der zugehörigen Figuren beschrieben.

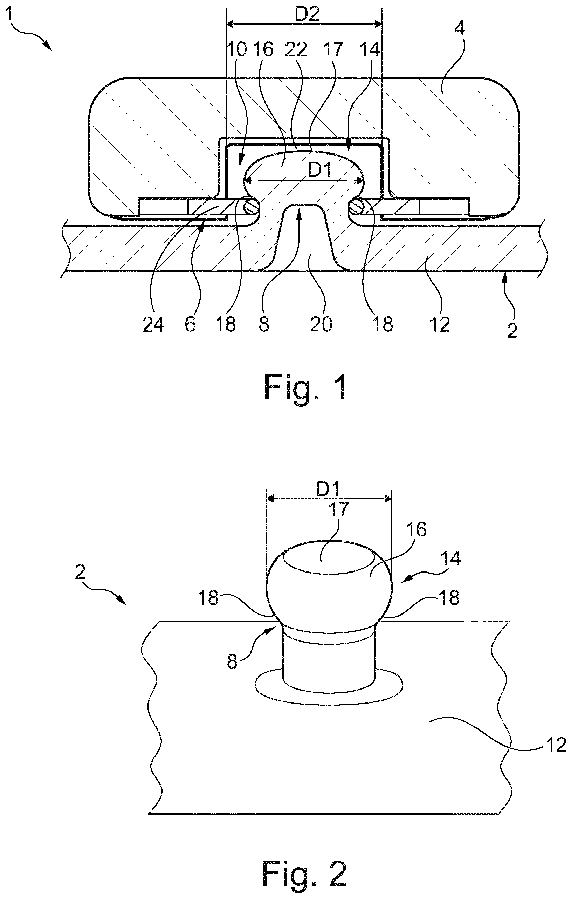

Figur 1 zeigt in einer Querschnittsansicht ein erfindungsgemäßes Behältersystem 1 einer ersten bevorzugten Ausführungsform. Das Behältersystem weist zwei separate Bauteile/Komponenten/Elemente auf, nämlich zum einen einen Behälter 2 in Form eines wannenförmigen Sterilcontainers oder eines wannenförmigen Siebkorbs oder eines Behälterdeckels und zum anderen ein daran anzukoppelndes Koppelement 4. In dieser Ausführungsform ist das Koppelelement 4 ein Kennzeichnungsschild, um den Inhalt des Behälters 2 anzugeben. Daneben existieren weitere Koppelelemente 4 in Form von Kennzeichnungsblenden und Perforationsabdeckungen für den Behälter 2.

Der Behälter 2 weist für eine Ankopplung des Koppelelements 4 ein Koppelsystem 6 mit einem männlichen Koppelabschnitt 8 und einem weiblichen Koppelabschnitt 10. Der männliche Koppelabschnitt 8 ist dabei an dem Behälter 2 ausgeformt, wohingegen der weibliche Koppelabschnitt 10 an dem Koppelelement 4 ausgebildet ist. Der männliche Koppelabschnitt 8 ist integral / stoffeinstückig in einer Behälterwand 12 des Behälters 2 ausgebildet und weist einen vorstehenden runden Knauf 14 bzw. einen runden Pilz 14 auf. Ein Kopf 16 des Knaufs 14 bildet dabei einen umfänglichen Hinterschnitt 18 aus. Der Knauf 14 weist in seiner Außenkontur die Form eines Pollers oder eines Sektkorkens auf.

An der Stelle des in der Behälterwand 12 ausgebildeten Knaufs 14 ist eine konkave, kapselförmige und rotationssymmetrische Aussparung bzw. Ausnehmung 20 innerhalb des Knaufs 14 ausgebildet. Der gegenüberliegende Kopf 16 ist in Figur 2 detailliert in einer perspektivische Ansicht des männlichen Koppelabschnitts 8 gezeigt. Der Knauf 14 ist rotationssymmetrisch ausgestaltet mit kreisrunder Kontur und weist einen sphärischen Kopf 16 auf. Der Kopf 16 ist an seiner Oberseite 17 abgeflacht. Der kreisrunde Kopf 16 mit einem Durchmesser D1, der den umfänglichen Hinterschnitt 18 ausbildet, greift in eine kreisrunde Vertiefung 22 in dem weiblichen Koppelabschnitt 10 mit einem Durchmesser D2 ein. Dabei ist der Durchmesser D1 des Knaufs 14 kleiner als der Durchmesser D2 der Vertiefung 22, so dass der Knauf 14 in diese einführbar ist. Ein Federelement 24 in Form eines Seegerrings mit runden Kanten ist in dem Koppelelement 4 eingefasst und umgreift / umfasst im angekoppelten Zustand den Kopf 16 des Knaufs 14 und damit den Hinterschnitt 18. Die Feder hat dabei einen Durchmesser, kleiner als der Durchmesser D1 des Kopfs 16. Durch eine Abrundung der Kanten des Hinterschnitts 18 bzw. einem gleichmäßigen Übergang des Kopfes 16 in Kombination mit dem abgerundeten Federelement, lässt sich ein Klickverschluss/Clipverschluss in Form eines Druckknopfsystems realisieren. Somit kann ein Anwender das Koppelelement 4 an den Behälter 2 mittels einer Druckkraft "aufklippen" (die Feder 24 wird entgegen ihrer Vorspannung auf einen Durchmesser größer als D1 aufgeweitet) und lösbar anbinden, als auch manuell durch eine Zugkraft (die Feder 24 wird abermals entgegen ihrer Vorspannung auf einen Durchmesser größer als D1 aufgeweitet) von dem Knauf 14 lösen. Dabei ist der Knauf 14 integral /stoffeinstückig in dem Behälter 2 ausgeformt, so dass keine Sterilbarriere durchbrochen wird und die Behälterwand 12 die natürliche undurchlässige Abgrenzung darstellt.

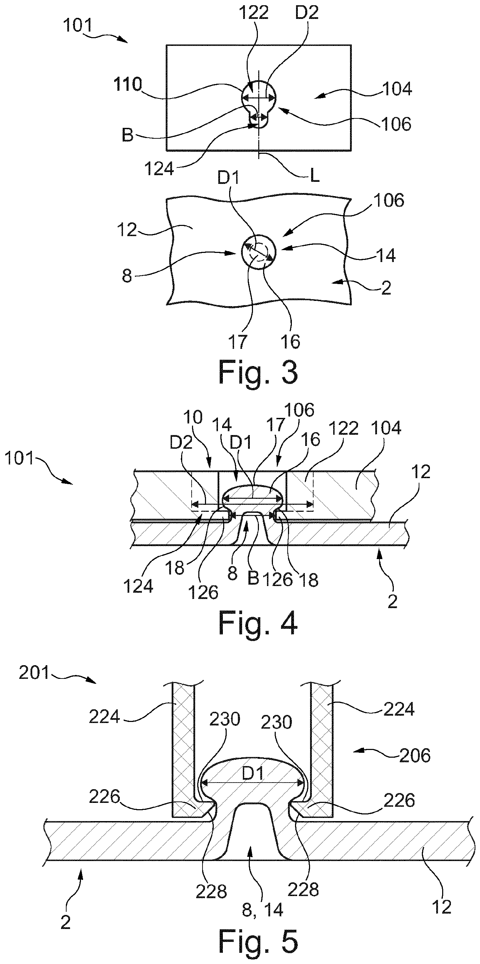

Figur 3 zeigt eine schematische Draufsicht eines erfindungsgemäßen Behältersystems 101 einer weiteren, zweiten bevorzugten Ausführungsform mit dem Behälter 2 und mit einem alternativen Koppelelement 104. In dieser Ausführungsform weist das Behältersystem 1 ein Kopplungssystem 106 mit zwar dem gleichen männlichen Koppelabschnitt 8 aber einem zu der ersten Ausführungsform unterschiedlich gestalteten weiblichen Koppelabschnitt 110 auf. Der weibliche Koppelabschnitt 110 weist in Draufsicht eine Form eines Schlüssellochs auf, mit einem kreisrunden Einlegeabschnitt 122 sowie einem daran direkt angrenzenden Rastabschnitt 124. Der Einlegeabschnitt 122 weist, wie auch schon in der ersten Ausführungsform einen Durchmesser D2 auf, der größer als der Durchmesser D1 des Kopfs 16 des Knaufs 14 ist. Der Rastabschnitt hingegen, in den der Einlegeabschnitt 122 übergeht, weist Führungsleisten 126 auf, die eine Breite B kleiner als der Durchmesser D1 des Kopfs 16 haben. Hierdurch wird der Kopf 16 in den beidseitigen Führungsleisten, zwar axialverschieblich in der Ausstreckung einer Längsachse L des Rastabschnitts 124 aber axialfest in einer Richtung quer dazu gefasst bzw. gehalten.

Figur 5 zeigt eine weitere alternative Ausführungsform eines erfindungsgemäßen Behältersystems 201 mit einem Koppelsystem 206. Der Behälter 2 mit dem männlichen Koppelabschnitt 8 ist wieder gleich zu den ersten beiden Ausführungsformen gehalten, wohingegen das Koppelelement 204 unterschiedlich ausgestaltet ist. Konkret weist das Koppelelement 204 einen alternativen weiblichen Arretierabschnitt 210 auf. Der weibliche Arretierabschnitt 210 ist in Form von zwei diametral gegenüberliegenden Rasthaken 224 ausgeformt. Die beiden Rasthaken 224 haben dabei aufeinander weisende Rastnasen 226, die auf einer Seite eine Rampe 228 und auf einer gegenüberliegenden Seite einen flachen Hinterschnitt 230 aufweisen, so dass sie in einer Richtung, unter elastischer Verformung des Rasthakens 224 mittels der Rampe 228 einfach auf den Knauf 14 aufgebracht werden können und diesen dann über den Hinterschnitt 230 in axialer Richtung formschlüssig halten, und in einer entgegengesetzten Richtung, unter erhöhtem Kraftaufwand (keine helfende Rampe 228) und erneuter elastischer Verformung wieder von dem Knauf 14 abgezogen werden können. Alternativ ist auch vorstellbar, dass sich die beiden diametral gegenüberliegenden Rasthaken 224 manuell gegeneinander verschieben lassen, so dass durch diese Verschiebung der Durchmesser erhöht wird und das Koppelelement 204 ohne elastische Verformung von dem Knauf 14 abgezogen werden kann.

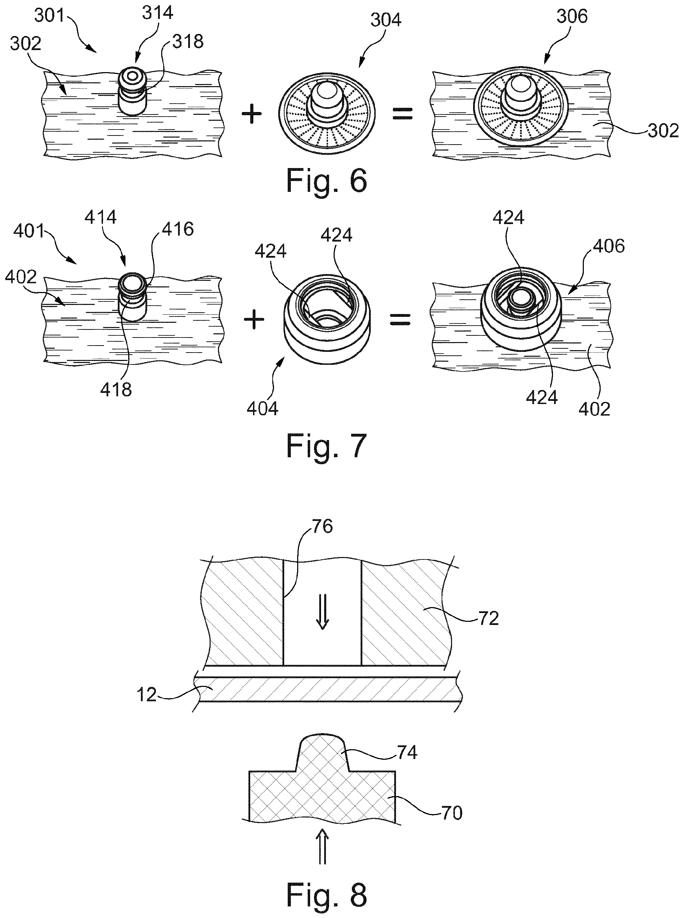

Figuren 6 und 7 zeigen jeweils ein weiteres Behältersystem 301 und 401 einer vierten und fünften bevorzugten Ausführungsform. In Figur 6 weist das Koppelelement 304 eine Hutform auf und wird, ähnlich zu der ersten Ausführungsform aus Figuren 1 und 2, auf den Knauf 314 aufgesteckt.

Figur 7 hingegen weist ein Koppelelement 404 in Form einer Buchse auf. Quer zu einer Längsachse der Buche verlaufen innerhalb der Buchse als weiblicher Koppelabschnitt 410 zwei parallele Federn 424. Die Federn 424 weisen dabei einen Abstand zueinander auf, der geringer ist als der Innendurchmesser der Buchse des weiblichen Koppelabschnitts 410. Diese Federn 424 halten den Hinterschnitt 418 des Knaufs 414 bzw. umgreifen / umfassen den Kopf 416 des Knaufs 414 federnd vorgespannnt. Damit kann im nicht angekoppelten Zustand der weibliche Koppelabschnitt 410 auf den männlichen Koppelabschnitt 408 mittels einer Druckkraft aufklippen und ankoppeln und im angekoppelten Zustand unter Aufbringung einer Zugkraft wieder entkoppeln.

Figuren 8 bis 11 zeigen die Schritte eines erfindungsgemäßen (Umform)Verfahrens einer bevorzugten Ausführungsform, um ein erfindungsgemäßes Behältersystem mit einem einstückig in dem Behälter 2 ausgebildeten Knauf 14, wie er in Fig. 1 gezeigt ist, herzustellen.

In Fig. 8 wird in einem ersten Schritt die Behälterwand 12 des Behälters 2, die insbesondere Aluminium als Material aufweist und in diesem Zeitpunkt noch plan / eben oder leicht gewölbt ist, positioniert und es werden bei einer vorbestimmten Stelle der Behälterwand 12, bei welcher zum Schluss der Knauf 14 ausgebildet werden soll, auf gegenüberliegenden Seiten einer Behälterwand 12 zwei komplementäre Pressmatrizen 70 und 72 koaxial zueinander angeordnet. Eine männliche Pressmatrize 70 der beiden Pressmatrizen weist dabei einen vollzylinderförmigen Vorsprung 74 mit abgerundeten Kanten und eine weibliche Pressmatrize 72 eine zu dem Vorsprung 74 komplementäre Matrizen-Vertiefung 76 in Form eines Hohlzylinders mit kreisrunder Durchgangsöffnung auf. Der Durchmesser des Vorsprungs 74 ist dabei geringer als der Durchmesser der Durchgangsöffnung der Matrizen-Vertiefung 76, sodass zwischen dem Vorsprung 74 und der Matrizen-Vertiefung 76 eine umfänglicher breiter Spalt verbleibt, der in etwa der Dicke der Behälterwand 12 an der vorbestimmten Stelle des Knaufs 14 entspricht. Alternativ kann der Spalt auch eine Dicke haben, welche gegenüber der Dicke der Behälterwand geringer ist. Insbesondere kann die Dicke des Spalts zwischen 5% und 90% der Dicke der Behälterwand betragen, besonders bevorzugt zwischen 10% und 70% und ganz besonders bevorzugt zwischen 20% und 40%.

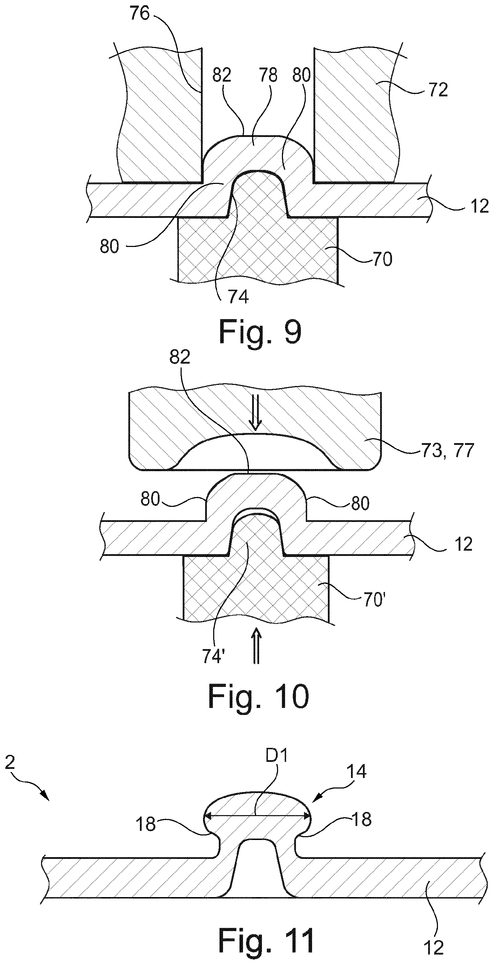

In einem zweiten Schritt werden dann die koaxial angeordneten komplementären Pressmatrizen 70, 72 gegeneinander bewegt, so dass diese zunächst auf die Behälterwand 12 aufdrücken bzw. die Spitze des Vorsprungs 74 und der stirnseitige Abschnitt des Hohlzylinder auf die Behälterwand 12 drücken und nach Erhöhung der Presskraft wird die Behälterwand 12 gepresst, so dass sich der umzuformende Bereich der Behälterwand 12 plastisch verformt und einen Napf 78 formt. Es wird sozusagen ein Auspressen bzw. Tiefziehen des Napfes 78 bewirkt. Dieser Zustand ist in Fig. 9 dargestellt. Das Material bzw. Blech der Behälterwand 12 passt sich also plastisch der Matrizenform der beiden komplementären Pressmatrizen 70, 72 an. Der Napf weist dabei eine kegelförmige oder zylindrische Umfangswand 80 und eine Stirnwand 82 auf. Nach diesem zweiten Schritt weist der Napf 78 allerdings noch keinen Hinterschnitt auf.

In einem dritten Schritt wird die männliche Pressmatrize 70 gegen eine weitere, unterschiedliche männliche Pressmatrize 70' ausgetauscht, die eine kürzeren Vorsprung 74' hat. Auch wird die weibliche Pressmatrize 72 gegen ein Werkzeug 73 mit sphärischer Matrize/Matrizenform/Stempelform 77 ausgetauscht. Dieser Zustand ist in Figur 10 gezeigt. Hierauf folgend wird die männliche Pressmatrize 72' wieder gegen das koaxial zu diesem angeordnete Werkzeug 73 bewegt und mit einer Presskraft gegeneinander gepresst. In Folge der Geometrien der Pressmatrizen bzw. des Werkzeugs wird der Napf 78 plastisch gestaucht bzw. der Napf 78 plastisch plattgedrückt und es formt sich ein Knauf 14 mit einem Kopf 16 und einem umlaufenden Hinterschnitt 18 aus, wie er in Fig. 11 zu sehen ist. Die männliche Pressmatrize 70 muss nicht zwangsläufig ausgewechselt werden. In einer nicht dargestellten Ausführungsform kann auch die männliche Pressmatrize 70 beibehalten werden und nur die weibliche Pressmatrize 72 getauscht werden.

Die weibliche Pressmatrize 72 ist in dieser Ausführungsform rohrförmig als Hohlzylinder mit einer Durchgangsbohrung ausgeführt. In einer alternativen Ausführungsform kann die weibliche Pressmatrize statt eines in Fig. 8 gesehen in Längsrichtung der weiblichen Pressmatrize 72 nach oben hin offenen Innenvolumens, in welches sich die zu pressende Behälterwand einfügt und in axialer Richtung der Pressmatrize somit keinen Anschlag ausbildet, auch eine nach oben hin geschlossene Stempelform, ähnlich zu dem Werkzeug 73, als Anschlag in axialer Richtung aufweisen, um gewissermaßen eine Vorformung des Kopfes des Knaufs zu erzielen.

In einer Ausführungsform kann der männliche Koppelabschnitt natürlich auch, statt mit einer zylindrischen oder kegelförmigen Umfangswand mit kreisförmiger Kontur bzw. kreisförmigen Querschnitt, mit einer mehreckigen Umfangswand mit mehreckiger Kontur, wie etwa einer fünfeckigen, einer sechseckigen, einer siebeneckigen oder einer achteckigen Kontur, ausgebildet sein. Alternativ kann die Umfangswand auch eine elliptische Kontur aufweisen. Insbesondere weisen auch die beiden Pressmatrizen eine mehreckige Kontur, bei der weiblichen Pressmatrize im Innenumfang und bei der männlichen Pressmatrize am Außenumfang des Vorsprungs, wie etwa einer fünfeckigen, einer sechseckigen, einer siebeneckigen oder einer achteckigen Kontur auf.

In einer weiteren Ausführungsform kann der rotationssymmetrisch gestaltete Knauf statt einem kreisrunden Querschnittsprofil mit einem sphärischen Kopf auch ein mehreckiges Profil, insbesondere ein sechseckiges oder ein achteckiges Profil des Kopfes aufweisen.

In einer Ausführungsform kann der weibliche Arretierungsabschnitt auch mehr als zwei Rasthaken, beispielsweise drei, vier, fünf oder sechs Rasthaken aufweisen.

In einer Ausführungsform kann der Behälter statt Aluminium natürlich auch Edelstahl als Material aufweisen.

Gemäß einer Ausführungsform kann eine männliche Pressmatrize der beiden Pressmatrizen einen vollzylinderförmigen Vorsprung statt abgerundete Kanten scharfe Kanten aufweisen, die beispielsweise mittels eines Stanzverfahrens hergestellt wurden, um das umzuformende Material des Behälters, insbesondere bei Materialien mit großer Dicke, für die weiteren Bearbeitungsschritte nach außen zu pressen.

### Bezugszeichenliste

- 1; 101; 201; 301; 401: Behältersystem
- 2; 302; 402: Behälter
- 4; 104; 204; 304; 404: Koppelelement
- 6; 106; 206; 306; 406: Koppelsystem
- 8: Männlicher Koppelabschnitt
- 10; 110; 210; 310; 410: Weiblicher Koppelabschnitt
- 12: Behälterwand
- 14; 314; 414: Knauf
- 16: Kopf
- 17: Kopf-Oberseite
- 18: Hinterschnitt
- 20: Ausnehmung
- 22: Vertiefung
- 24: Feder
- 70; 70': Männliche Pressmatize
- 72: Weibliche Pressmatrize
- 73: Werkzeug
- 74; 74': Vorsprung
- 76: Matrizen-Vertiefung
- 77: Sphärische Matrize
- 78: Napf
- 80: Umfangswand
- 82: Stirnwand
- 122: Einlegeabschnitt
- 124: Rastabschnitt
- 126: Führungsleiste
- 224: Rasthaken
- 226: Rastnase
- 424: Feder

- L: Längsachse Rastabschnitt
- B: Breite
- D1: Durchmesser Kopf
- D2: Durchmesser Vertiefung

## Patentansprüche

1. Behältersystem (1; 101; 201; 301; 401) mit einem Behälter (2), insbesondere medizinischer oder chirurgischer Sterilbehälter vorzugsweise mit einem wannenartigen Behältergefäß und/oder einem Behälterdeckel, mit zumindest einem separaten Koppelelement (4) und mit zumindest einem Koppelsystem (6) zur lösbaren Anbindung des Koppelelements (4) an den Behälter (2), wobei das Koppelsystem (6) einen an dem Behälter (2) ausgebildeten männlichen Koppelabschnitt (8) und einen an dem Koppelelement (4) ausgebildeten weiblichen Koppelabschnitt (10; 110; 210; 310; 410) aufweist, die miteinander koppelbar und entkoppelbar sind, wobei der männliche Koppelabschnitt (8) integral in einer Wand (12) des Behälters (2) ausgebildet ist, **dadurch gekennzeichnet, dass** der männliche Koppelabschnitt (8) in Form eines vorstehenden Knaufs (14) mit einer pilzförmigen Struktur mit einem Hinterschnitt (18) gestaltet ist.

2. Behältersystem (1) nach Anspruch 1, **dadurch gekennzeichnet, dass** das Koppelsystem (6) in Form eines Druckknopfsystems ausgebildet ist, bei dem der weibliche Koppelabschnitt (10) eine kreisrunde Vertiefung (22) aufweist, in welche der Knauf (14) in einem angekoppelten Zustand hineinsteht, wobei der Hinterschnitt (18) des Knaufs (14) durch den weiblichen Koppelabschnitt (10) vorgespannt umgriffen wird.

3. Behältersystem (1) nach Anspruch 2, **dadurch gekennzeichnet, dass** der weibliche Koppelabschnitt (10) einen umfänglichen Hinterschnitt aufweist, der den Hinterschnitt (18) des Knaufs (14) umgreift und mittels einer Materialelastizität den Knauf (14) lösbar ankoppelt oder dass der weibliche Koppelabschnitt (10) eine Feder (24) aufweist, die den Hinterschnitt (18) des Knaufs (14) hintergreift, um den Knauf (14) lösbar anzukoppeln.

4. Behältersystem (101) nach Anspruch 1, **dadurch gekennzeichnet, dass** der weibliche Koppelabschnitt (101) eine Vertiefung in Form eines Schlüssellochs mit einem runden Einlegeabschnitt (122) und einem Rastabschnitt (124) mit parallelen Hinterschnitten in Form von Führungsleisten (126) aufweist, die derart ausgestaltet ist, dass der Knauf (14) in dem Einlegeabschnitt (122) aufnehmbar und entnehmbar ist und nach einer Verschiebung des Knaufs (14) in den Rastabschnitt (124) über die Führungsleisten (126) formschlüssig angekoppelt ist.

5. Behältersystem (201) nach Anspruch 1, **dadurch gekennzeichnet, dass** der weibliche Koppelabschnitt (210) in Form von zwei gegenüberliegenden Rastnasen (224) ausgebildet ist, welche im angekoppelten Zustand den Knauf (14) umgreifen und den männlichen Koppelabschnitt (8) formschlüssig ankoppeln.

6. Behältersystem (1; 101; 201) nach einem der vorangegangenen Ansprüche, **dadurch gekennzeichnet, dass** der Behälter (2) ein Sterilcontainer ist und das Koppelelement (4; 104; 204) ein Kennzeichnungsschild oder eine Kennzeichnungsblende oder eine Perforationsfeldabdeckung oder ein Sensor oder ein Identifizierungselement oder ein Halteelement ist.

7. Verfahren zur Ausbildung eines Knaufs (14) in einem Behälter (2) für ein Behältersystem, insbesondere einem medizinischen oder chirurgischen Sterilbehälter vorzugsweise mit einem wannenartigen Behältergefäß und/oder einem Behälterdeckel, **gekennzeichnet durch die Schritte:**
Anordnen von zwei komplementären Pressmatrizen (70, 72) auf gegenüberliegenden Seiten einer Wand (12) des Behälters (2), wobei eine männliche Pressmatrize (70) der beiden Pressmatrizen einen Vorsprung (74) und eine weibliche Pressmatrize (72) eine komplementäre Matrizen-Vertiefung (76) aufweist;
Pressen der komplementären Pressmatrizen (70, 72) gegeneinander und damit Auspressen / Tiefziehen eines Napfes (78); und
Stauchen des Napfes (78), so dass ein Knauf (14) mit einer pilzförmigen Struktur mit einem Kopf (16) und einem umlaufenden Hinterschnitt (18) ausgeformt wird.

8. Verfahren nach Anspruch 7, **dadurch gekennzeichnet, dass** der Schritt Stauchen des Napfes (78) durch ein Werkzeug (73) mit einer planen oder sphärischer und vorzugsweise rotationssymmetrischen Matrize (77) erfolgt, welche den Napf (78) plattdrückt und durch plastische Verformung den Hinterschnitt (18) formt.

9. Verfahren nach Anspruch 7, **dadurch gekennzeichnet, dass** der Schritt Stauchen des Napfes (78) die Schritte aufweist:
Wechsel der weiblichen Pressmatrize (72) mit der männlichen Pressmatrize (70) ; und
Pressen der komplementären Pressmatrizen (70, 72) gegeneinander.

10. Verfahren nach Anspruch 7, **dadurch gekennzeichnet, dass** der Schritt Stauchen des Napfes (78) die Schritte aufweist:
Pressen des Napfes (78) durch ein Formwerkzeug mit einer definierten hinterschnittigen Knauf-Matrizenkontur, in welches sich der Napf (78) plastisch einpresst und anformt und den Knauf (14) ausbildet; und
Teilen des Formwerkzeugs und entformen des Knaufs (14).

## Claims

1. A container system (1; 101; 201; 301; 401) comprising a container (2), in particular a medical or surgical sterile container preferably having a tub-like container vessel and/or a container cover, at least one separate coupling element (4) and at least one coupling system (6) for releasably connecting the coupling element (4) to the container (2), wherein the coupling system (6) includes a male coupling portion (8) formed on the container (2) and a female coupling portion (10; 110; 210; 310; 410) formed on the coupling element (4), wherein the coupling portions can be coupled to and uncoupled from one another, wherein the male coupling portion (8) is integrally formed in a wall (12) of the container (2) **characterized in that** the male coupling portion (8) is designed in the form of a protruding knob (14) with a mushroom-shaped structure with an undercut (18).

2. The container system (1) according to claim 1, **characterized in that** the coupling system (6) is formed in the form of a push-button system in which the female coupling portion (10) has a circular depression (22) into which the knob (14) protrudes in a coupled state, the undercut (18) of the knob (14) being encompassed by the female coupling portion (10) in a prestressed manner.

3. The container system (1) according to claim 2, **characterized in that** the female coupling portion (10) has a circumferential undercut which encompasses the undercut (18) of the knob (14) and releasably couples the knob (14) by means of material elasticity, or the female coupling portion (10) has a spring (24) which reaches behind the undercut (18) of the knob (14) in order to releasably couple the knob (14).

4. The container system (101) according to claim 1, **characterized in that** the female coupling portion (101) has a recess in the form of a keyhole with a round insert section (122) and a detent section (124) with parallel undercuts in the form of guide bars (126), which is configured such that the knob (14) can be received in and removed from the insert section and, after shifting the knob (14) into the detent section (124), is positively coupled via the guide bars (126).

5. The container system (201) according to claim 1, **characterized in that** the female coupling portion (210) is formed in the form of two diametrically opposed snap-in noses (224) which, in a coupled state, encompass the knob (14) and thus positively couple the male coupling portion (8).

6. The container system (1; 101; 201) according to any of the preceding claims, **characterized in that** the container (2) is a sterile container and the coupling element (4; 104; 204) is an identification label or an identification bezel or a perforation field cover or a sensor or an identification element or a holding element.

7. A method for forming a knob (14) in a container (2) for a container system, in particular a medical or surgical sterile container, preferably including a tub-like container vessel and/or a container cover,
**characterized by the following steps:**
arranging two complementary press dies (70, 72) on opposite sides of a wall (12) of the container (2), a male press die (70) of the two press dies having a protrusion (74), and a female press die (72) having a complementary die recess (76);
pressing the complementary press dies (70, 72) against each other and thereby squeezing / deep-drawing a cup (78); and
compressing the cup (78) so that a knob (14) with a mushroom-shaped structure is formed with a head (16) and a circumferential undercut (18).

8. The method according to claim 7, **characterized in that** the step of compressing the cup (78) is performed by a tool (73) with a planar or spherical and preferably rotationally symmetrical die (77) that flattens the cup (78), thereby forming the undercut (18) by plastic deformation.

9. The method according to claim 7, **characterized in that** the step of compressing the cup (78) includes the following steps:
alternating the female press die (72) and the male press die (70); and
pressing the complementary press dies (70, 72) against each other.

10. The method according to claim 7, **characterized in that** the step of compressing the cup (78) includes the following steps:
pressing the cup (78) by way of molding tool having a defined undercut knob die contour into which the cup (78) is pressed plastically and is preformed, thus forming the knob (14); and
splitting the molding tool and deforming the knob.

## Revendications

1. Système de récipient (1 ; 101 ; 201 ; 301 ; 401) avec un récipient (2), en particulier un récipient stérile médical ou chirurgical de préférence avec une enveloppe de récipient à cuve et/ou un couvercle de récipient, avec au moins un élément de couplage (4) séparé et avec au moins un système de couplage (6) pour la liaison amovible de l'élément de couplage (4) avec le récipient (2), dans lequel le système de couplage (6) présente une section de couplage (8) mâle formée au niveau du récipient (2) et une section de couplage (10; 110; 210; 310; 410) femelle formée au niveau de l'élément de couplage (4) qui peuvent être couplées et découplées l'une avec l'autre, dans lequel la section de couplage (8) mâle est formée d'un seul tenant dans une paroi (12) du récipient (2), **caractérisé en ce que** la section de couplage (8) mâle est conçue sous la forme d'une poignée (14) en saillie avec une structure en forme de champignon avec une contre-dépouille (18).

2. Système de récipient (1) selon la revendication 1, **caractérisé en ce que** le système de couplage (6) est formé sous la forme d'un système de bouton pression, pour lequel la section de couplage (10) femelle présente une cavité (22) circulaire, dans laquelle la poignée (14) se trouve dans un état couplé, dans lequel la contre-dépouille (18) de la poignée (14) est entourée de manière précontrainte par la section de couplage (10) femelle.

3. Système de récipient (1) selon la revendication 2, **caractérisé en ce que** la section de couplage (10) femelle présente une contre-dépouille périphérique qui entoure la contre-dépouille (18) de la poignée (14) et couple de manière amovible la poignée (14) au moyen d'une élasticité de matériau ou **en ce que** la section de couplage (10) femelle présente un ressort (24) qui vient en prise derrière la contre-dépouille (18) de la poignée (14) afin de coupler de manière amovible la poignée (14).

4. Système de récipient (101) selon la revendication 1, **caractérisé en ce que** la section de couplage (101) femelle présente une cavité en forme de serrure avec une section d'introduction (122) ronde et une section d'encliquetage (124) avec des contre-dépouilles parallèles en forme de baguettes de guidage (126), cavité qui est configurée de telle manière que la poignée (14) puisse être reçue et retirée dans la section d'introduction (122) et
est couplée par complémentarité de formes après un déplacement de la poignée (14) dans la section d'encliquetage (124) par le biais des baguettes de guidage (126).

5. Système de récipient (201) selon la revendication 1, **caractérisé en ce que** la section de couplage (210) femelle est formée sous la forme de deux nez d'encliquetage (224) opposés qui entourent dans l'état couplé la poignée (14) et couplent par complémentarité de formes la section de couplage (8) mâle.

6. Système de récipient (1 ; 101 ; 201) selon l'une quelconque des revendications précédentes, **caractérisé en ce que** le récipient (2) est un contenant stérile et l'élément de couplage (4 ; 104 ; 204) est une plaque d'identification ou un panneau d'identification ou un recouvrement de champ de perforation ou un capteur ou un élément d'identification ou un élément de retenue.

7. Procédé de réalisation d'une poignée (14) dans un récipient (2) pour un système de récipient, en particulier un récipient stérile médical ou chirurgical de préférence avec une enveloppe de récipient à cuve et/ou un couvercle de récipient, **caractérisé par les étapes suivantes** :
l'agencement de deux matrices de presse (70, 72) complémentaires sur des côtés opposés d'une paroi (12) du récipient (2), dans lequel une matrice de presse (70) mâle des deux matrices de presse présente une saillie (74) et une matrice de presse (72) femelle présente une cavité de matrice (76) complémentaire ;
le pressage des matrices de presse (70, 72) complémentaires l'une contre l'autre et ainsi le pressage/l'emboutissage d'un godet (78) ; et
le refoulement du godet (78) de sorte qu'une poignée (14) soit moulée avec une structure en forme de champignon avec une tête (16) et une contre-dépouille (18) tournante.

8. Procédé selon la revendication 7, **caractérisé en ce que** l'étape de refoulement du godet (78) est effectuée par un outil (73) avec une matrice (77) plane ou sphérique et de préférence à symétrie de rotation qui écrase le godet (78) et forme la contre-dépouille (18) par déformation plastique.

9. Procédé selon la revendication 7, **caractérisé en ce que** l'étape de refoulement du godet (78) présente les étapes suivantes :
le remplacement de la matrice de presse (72) femelle par la matrice de presse (70) mâle ;
le pressage des matrices de presse (70, 72) complémentaires l'une contre l'autre.

10. Procédé selon la revendication 7, **caractérisé en ce que** l'étape de refoulement du godet (78) présente les étapes suivantes :
le pressage du godet (78) par un outil de formage avec un contour de matrice de poignée contre-dépouillé défini, dans lequel le godet (78) s'enfonce et se forme plastiquement et forme la poignée (14) ; et
la séparation de l'outil de formage et le démoulage de la poignée (14).
